# EUROPÄISCHE PATENTSCHRIFT

(11) **EP 0 631 577 B1**
(45) Veröffentlichungstag und Bekanntmachung des Hinweises auf die Patenterteilung: **28.05.1997**
(21) Anmeldenummer: 93906485.3
(22) Anmeldetag: 05.03.1993
(51) Int. Cl.: C07D 311/72

(54) **VERFAHREN ZUR HERSTELLUNG VON VITAMIN E**
PROCESS FOR PRODUCING VITAMIN E
PROCEDE DE FABRICATION DE VITAMINE E

(30) Priorität: 17.03.1992 DE 4208477
(43) Veröffentlichungstag der Anmeldung: 04.01.1995
(73) Patentinhaber: BASF Aktiengesellschaft, 67063 Ludwigshafen (DE)
(72) Erfinder: GRAFEN, Paul, D-6719 Weisenheim (DE); KIEFER, Hans, D-6730 Neustadt (DE); JAEDICKE, Hagen, D-6700 Ludwigshafen (DE)
(86) Internationale Anmeldenummer: EP9300498
(87) Internationale Veröffentlichungsnummer: WO9319057

(56) Entgegenhaltungen:
- EP-A- 0 100 471
- DE-A- 2 404 621
- DE-C- 1 015 446

## Beschreibung

Die Erfindung betrifft ein verbessertes Verfahren zur Herstellung von Vitamin E durch säurekatalysierte Umsetzung von 2,3,5-Trimethylhydrochinon (TMH) mit einem Phytol.

Es ist bereits bekannt dl-α-Tocopherol (Vitamin E) durch Umsetzen von TMH und einem Phytol, beispielsweise Isophytol (IP) bei erhöhter Temperatur in einem wenig polaren Lösungsmittel in Gegenwart verschiedener saurer Katalysatoren herzustellen.

Nach Angaben in Chem. Abstracts. (C.A.), 84 (1976), 59792 und C.A., 85 (1976), 46898 kann die Umsetzung in Gegenwart von SiO₂/Al₂O₃ mit Säuren durchgeführt werden.

Gemäß C.A., 73 (1970), 77483; C.A., 80 (1974) 3385: C.A., 80 (1974), 3386; C.A., 73 (1970), 98799 und DE-OS 22 08 795 kann die Umsetzung auch in Gegenwart von ZnCl₂ in Kombination mit Säuren, wie Halogenwasserstoffsäuren, insbesondere HCl, Trichloressigsäure oder Essigsäure durchgeführt werden.

Gemäß den Angaben in DE-A-22 08 795 ist auch ZnCl₂ im Gemisch mit NaHSO₄, Schwefelsäure oder p-Toluolsulfonsäure in einem Molverhältnis von 1:3 bis 1:1 verwendbar.

Nach den Angaben in C.A., 84 (1976), 74471 ist die Umsetzung in Gegenwart eines Gemisches aus SiO₂ und Al₂O₃ im Verhältnis 87:13 als Katalysator in Perchlorethylen durchführbar.

Gemäß US 3 459 773 erhält man Vitamin-E durch Umsetzen von Phytol oder Isophytol mit TMH in einem inerten Lösungsmittel unter Verwendung eines makroretikularen Kationenaustauscherharzes vom Sulfonsäuretyp.

Allen diesen bekannten Verfahren ist gemeinsam, daß das Vitamin E in großtechnischem Maßstab nicht in der erforderlichen Reinheit hergestellt werden kann.

Zur Überwindung dieses Problems wurde die Umsetzung gemäß DE 27 43 920 in Gegenwart von einem Gemisch aus Kieselsäure/ Aluminiumoxid oder Kieselsäuregel und Zinkchlorid sowie einer starken Säure, wie konzentrierter HCl, H₂SO₄, H₃PO₄ oder p-Toluolsulfonsäure durchgeführt.

Nachteilig an diesem Verfahren ist - wie bei zahlreichen der oben genannten Verfahren auch - daß Korrosionsprobleme auftreten und daß eine Belastung des Abwassers mit Zinkionen auftreten kann.

Es war daher die Aufgabe der Erfindung, ein Katalysatorsystem für die Umsetzung von TMH und Phytol oder Isophytol zu Vitamin E zu finden, mit dessen Hilfe die Herstellung von Vitamin E auch in größerem Maßstab vorteilhafter gelingt als gemäß dem Stand der Technik. Hierzu war es nötig, daß das Katalysatorsystem nicht toxisch ist (wie BF₃-Addukte), daß es nicht korrosiv wirkt (wie ZnCl₂, HCl, HCOOH und H₃PO₄), daß es unerwünschte Nebenreaktionen, wie die Dehydratisierung des Isophytols zu Phytadienen, möglichst weitgehend unterdrückt und daß es eine Umsetzung der teuren und empfindlichen Ausgangsmaterialien auch bei Einsatz äquimolarer Mengen in hoher Ausbeute katalysiert, denn die überschüssigen Ausgangsmaterialien sind nur mit großem Aufwand vom Reaktionsprodukt abtrennbar.

Es wurde nun überraschenderweise gefunden, daß eine Mischung aus ortho-Borsäure einerseits und bestimmten Dicarbonsäuren, wie Oxalsäure oder Weinsäure oder einer Tricarbonsäure, wie Citronensäure, andererseits, insbesondere eine Mischung aus ortho-Borsäure und Oxalsäure, die genannten Anforderungen mit Abstand am besten erfüllt. Jede Komponente allein katalysiert die Umsetzung nur unzureichend. Erst der unerwartete synergistische Effekt ermöglicht das optimale Ergebnis.

Gegenstand der Erfindung ist dementsprechend ein verbessertes Verfahren zur Herstellung von dl-α-Tocopherol bzw. dl-α-Tocopherylacetat durch säurekatalysierte Umsetzung von 2,3,5-Trimethylhydrochinon mit Phytol oder Isophytol in einem Lösungsmittel bei erhöhter Temperatur und ggf. anschließende Veresterung des erhaltenen Tocopherols mit Acetanhydrid, das dadurch gekennzeichnet ist, daß man die Umsetzung in Gegenwart von einer Mischung aus ortho-Borsäure einerseits und Oxalsäure, Weinsäure oder Citronensäure andererseits durchführt.

Die Herstellung von TMH und Phytol bzw. Isophytol ist bekannt und braucht daher nicht erörtert zu werden.

Besonders vorteilhaft gestaltet sich das Verfahren, wenn man Oxalsäure oder Weinsäure, insbesondere Oxalsäure im Gemisch mit ortho-Borsäure als Katalysator verwendet.

Die Kondensation von TMH mit Phytol oder Isophytol wird erfindungsgemäß in Gegenwart von etwa 0,2 bis 7 mol%, vorzugsweise 0,5 bis 5 mol%, ortho-Borsäure und 0,4 bis 14 mol%, vorzugsweise 1 bis 10 mol%, Oxalsäure, Weinsäure oder Citronensäure pro mol TMH durchgeführt. Die ortho-Borsäure und die Carbonsäure verwendet man mit Vorteil etwa im molaren Verhältnis von 1:2.

Als Lösemittel für die Umsetzung werden mit Vorteil Alkylaromaten, wie Toluol oder ein Xylol oder aber Ketone mit einem Siedepunkt zwischen etwa 70 und 140°C eingesetzt. Mit besonderem Vorteil arbeitet man in aliphatischen Ketonen, wie Diethylketon oder Methylisopropylketon. Recht gut geeignet sind aber auch höhersiedende Lösungsmittel wie Tetralin, wenn man die Kondensation unter stark vermindertem Druck durchführt.

Die Reaktionstemperaturen betragen im allgemeinen etwa 70 bis 130°C, vorzugsweise 90 bis 110°C.

Zur Durchführung der Reaktion geht man im allgemeinen so vor, daß man zu einer Lösung von TMH und dem Katalysatorgemisch in dem Lösemittel unter Trocknen des Reaktionsgemisches durch Wasserauskreisen und unter Rückflußkühlung das Phytol oder Isophytol langsam zufügt. Nach beendeter Umsetzung (Bestimmung durch HPLC) wird in an sich bekannter Weise aufgearbeitet. Bei Verwendung von Ketonen als Lösemittel muß dieses nach der Reaktion entfernt und durch ein nicht mit Wasser mischbares Lösemittel, insbesondere einen Kohlenwasserstoff, ersetzt werden, damit das erhaltene Roh-Tocopherol gewaschen werden kann. Bei Verwendung von Alkylaromaten als Lösemittel kann ein Lösemittelaustausch entfallen. Dafür hat die Verwendung von Alkylaromaten als Lösemittel den Nachteil, daß der Katalysator in geringen Mengen aus dem Reaktionsgemisch raussublimiert.

Das Waschen des Roh-Tocopherols kann beispielsweise mit verdünnter wäßriger NaOH-Lösung, mit einer Mischung aus Methanol und verdünnter wäßriger HCl und anschließend einer Mischung aus Methanol und einer verdünnten wäßrigen Natriumbicarbonatlösung erfolgen. Das so erhaltene Tocopherol kann entweder als solches isoliert oder aber mit überschüssigem Acetanhydrid säurekatalysiert in Tocopherylacetat überführt werden.

Das so erhaltene Tocopherylacetat kann durch fraktionierende Destillation unter stark vermindertem Druck gereinigt werden.

Das Verfahren kann diskontinuierlich, aber auch kontinuierlich durchgeführt werden.

Mit Hilfe des erfindungsgemäßen Verfahrens erhält man dl-α-Tocopherol bzw. sein Acetat auf einfache und umweltschonende Weise in sehr guter Ausbeute und Reinheit.

### Beispiel 1

In einem 1 l-Kolben wurden 76 g (0,5 mol) TMH mit 0,973 g (15,7 mmol) ortho-Borsäure und 2,83 g (31,4 mmol) Oxalsäure in 200 g (245,2 ml) Diethylketon vorgelegt. Das Reaktionsgemisch wurde unter Trocknen durch Wasserauskreisen innerhalb von 3 Stunden (h) unter intensiver Rückflußkühlung tropfenweise mit 160 g (0,535 mol) Isophytol versetzt.

Nach beendeter Zugabe von Isophytol wurde das Reaktionsgemisch noch 30 Minuten (min) unter Rückfluß zum Sieden erhitzt und dann abkühlen lassen. Mittels HPLC (100 % Methanol, Zorbax ODS 5 µm, 4 mm x 25 mm, 1 ml/min, UV-Detektor bei. 220 nm) wurde der Umsatz an TMH als >96 % bestimmt.

Anschließend wurde mit verdünnter wäßriger NaOH-Lösung das unumgesetzte TMH und der Katalysator aus der erhaltenen Tocopherol-Lösung extrahiert und die Tocopherol-Lösung mit einer wäßrigen alkalischen Natriumsulfitlösung entfärbt.

Dann wurde das Diethylketon unter vermindertem Druck abdestilliert und der verbleibende Rückstand mit 66 g (0,646 mol) Essigsäureanhydrid und 1,5 ml einer Lösung von 1 ml H₂SO₄ in 100 ml Essigsäureanhydrid versetzt und (unter HPLC-Kontrolle) 1 h unter Rückfluß zum Sieden erhitzt.

Anschließend wurde das Essigsäureanhydrid-Essigsäuregemisch bei 20 mbar abdestilliert und durch Destillation unter vermindertem Druck 224 g reines Tocopherylacetat gewonnen. Das entspricht einer Ausbeute von 91,7 % der Theorie.

### Beispiele 2 bis 4

Eine Mischung aus 22,8 g (0,15 mol) TMH, 9,4 mmol der aus der Tabelle ersichtlichen Carbonsäure und 0,293 g (4,7 mmol) an ortho-Borsäure in 70 ml Diethylketon wurde unter Trocknen durch Wasserauskreisen innerhalb von 1,5 h unter intensiver Rückflußkühlung tropfenweise mit 48 g (0,16 mol) Isophytol versetzt. Anschließend wurde das Reaktionsgemisch noch 2 h unter Rückfluß zum Sieden erhitzt. Während der Umsetzung wurden ca. 2 ml Wasser ausgekreist.

Anschließend wurde das Diethylketon unter vermindertem Druck (60°C Badtemperatur, Wasserstrahlpumpe, Rotationsverdampfer) abdestilliert, der Rückstand in 100 ml Heptan aufgenommen und die Heptanlösung zweimal mit je 150 ml eines 1:1-Gemisches aus Methanol und 1 n Salzsäure, dann einmal mit einem Gemisch aus 150 ml Methanol und 150 ml einer 2,5 %igen wäßrigen Natriumbicarbonat-Lösung und schließlich einmal mit 75 ml eines 1:1-Gemisches von Methanol und Wasser gewaschen.

Durch Abdampfen des Heptans unter vermindertem Druck (70°C Badtemperatur, Wasserstrahlpumpe, Rotationsverdampfer) wurde roh-Tocopherol erhalten.

Das Roh-Tocopherol wurde mit 19,2 g (0,19 mol) Acetanhydrid und einer katalytischen Menge Schwefelsäure acetyliert (142-145°C; 4 h) und das Reaktionsgemisch anschließend unter vermindertem Druck eingeengt. Das so erhaltene rohe Tocopherylacetat wurde durch fraktionierende Destillation unter stark vermindertem Druck gereinigt und gaschromatographisch die Ausbeute bestimmt. Die erzielten Ausbeuten an α-Tocopherylacetat sind in der folgenden Tabelle dargestellt.

**Tabelle**

| Beispiel | Carbonsäure | Ausbeute an α-Tocopherylacetat [% d.Th.] |
|---|---|---|
| 2 | Oxalsäure | 95 |
| 3 | L-(+)-Weinsäure | 85 |
| 4 | Citronensäure | 69 |

### Beispiel 5

a) In einer 500 ml-Rührapparatur mit Thermometer, Tropftrichter und einer Vorrichtung zum Auskreisen von Wasser wurde eine Mischung aus 150 ml Toluol, 22,8 g (0,15 mol) TMH, 0,29 g (5 mmol) ortho-Borsäure und 0,85 g (10 mmol) Oxalsäure vorgelegt. Diese Mischung wurde innerhalb von 1 h unter intensiver Rückflußkühlung (ca. 100°C/650 mbar) und Trocknen durch Wasserauskreisen tropfenweise mit 48 g (0,16 mol) Isophytol versetzt und noch 1 h nachreagieren lassen. Etwa 2,4 ml Wasser wurden ausgekreist.
   Anschließend wurde das Reaktionsgemisch auf Raumtemperatur abgekühlt und 3 mal mit je 150 ml einer Mischung aus gleichen Teilen Methanol und 1 m wäßriger HCl und dann 2 mal mit je 150 ml an einem 50 %igen wäßrigen Methanol gewaschen und schließlich am Rotationsverdampfer (65°C Badtemperatur/30 mbar) eingeengt.
   Zu dem erhaltenen roh-Tocopherol wurden dann 19,2 g (0,19 mol) Acetanhydrid und eine Spur Schwefelsäure zugefügt, das Gemisch 4 h unter Rückfluß zum Sieden erhitzt und anschließend am Rotationsverdampfer (65°C Badtemperatur/30 mbar) eingeengt. Das so erhaltene rohe Tocopherylacetat wurde durch fraktionierende Destillation unter stark vermindertem Druck gereinigt. Bei 200 bis 210°C/0,01 mbar gingen 65,2 g α-Tocopherylacetat über, die nach gaschromatographischer Analyse (GC) eine Reinheit von 98 % aufwiesen. Dies entspricht einer Ausbeute von 90 % der Theorie.
   Die erzielbaren Ausbeuten können in geringem Umfang verbessert werden durch Variation der Aufarbeitung des Reaktionsgemisches. Folgende Aufarbeitungsvariante b) wurde durchgeführt.
b) Es wurde gearbeitet wie in a) beschrieben, jedoch wurde nach der Reation + Nachreaktion mit Isophytol das Toluol abdestilliert (Rotationsverdampfer, 65°C, 30 mbar) und durch die gleiche Menge n-Hexan ersetzt, das Reaktionsgemisch wie unter a) beschrieben gewaschen und dann das n-Hexan mit vermindertem Druck abdestilliert.
   Das durch fraktionierende Destillation erhaltene α-Tocopherylacetat hatte gemäß GC eine Reinheit von 98 %. Ausbeute 92 % der Theorie.

### Beispiel 6

In einer 500 ml-Rührapparatur mit Thermometer und Tropftrichter wurde eine Mischung aus 150 ml Tetralin, 22,8 g TMH, 0,29 g ortho-Borsäure und 0,85 g Oxalsäure vorgelegt. Diese Mischung wurde innerhalb von 1 h unter intensiver Rückflußkühlung (95 bis 100°C bei ca. 80 mbar) tropfenweise mit 48 g Isophytol versetzt und bei dieser Temperatur noch 1 h nachreagieren lassen. Das Reaktionswasser wurde während der Umsetzung dampfförmig über eine beheizte Brücke abgezogen und in einer gekühlten Vorlage kondensiert (ca. 2,6 g). Dabei wurde auch ein geringer Teil des Katalysators mitgerissen.

Anschließend kühlte man auf 40°C ab, setzte 100 ml Heptan zu und wusch dreimal mit je 150 ml einer Lösung aus gleichen Teilen Methanol und 1 m wäßriger Salzsäure und anschließend zweimal mit je 100 ml an 50 %igem wäßrigem Methanol. Die organische Phase wurde eingeengt indem man das Heptan am Rotationsverdampfer abzog (65°C Badtemperatur/ca. 30 mbar) und Tetralin über eine Brücke abdestillierte (50°C/ca. 0,5 mbar).

Zum Rückstand wurden 19,2 g Acetanhydrid und eine Spur Schwefelsäure addiert, das Gemisch 4 h auf Rückflußtemperatur erhitzt und dann am Rotationsverdampfer (65°C Badtemperatur, 30 mbar, zuletzt 5 mbar) eingeengt. Destillation unter stark vermindertem Druck über eine Brücke lieferte bei 204 bis 210°C/0,02 mbar 68,6 g α-Tocopherylacetat, das nach GC eine Reinheit von 92 % aufweist; dies entspricht einer Ausbeute von 89 % der Theorie.

## Patentansprüche

1. Verfahren zur Herstellung von dl-α-Tocopherol bzw. dl-α-Tocopherylacetat durch säurekatalysierte Umsetzung von 2,3,5-Trimethyl-hydrochinon mit Phytol oder Isophytol in einem Lösungsmittel bei erhöhter Temperatur und ggf. anschließende Veresterung des erhaltenen Tocopherols mit Acetanhydrid, dadurch gekennzeichnet, daß man die Umsetzung in Gegenwart von einer Mischung aus ortho-Borsäure einerseits und Oxalsäure, Weinsäure oder Citronensäure andererseits durchführt.

2. Verfahren zur Herstellung von dl-α-Tocopherol bzw. dl-α-Tocopherylacetat gemäß Anspruch 1, dadurch gekennzeichnet, daß man die Umsetzung in Gegenwart von einer Mischung aus ortho-Borsäure und Oxalsäure durchführt.

3. Verfahren zur Herstellung von dl-α-Tocopherol bzw. dl-α-Tocopherylacetat gemäß Anspruch 1, dadurch gekennzeichnet, daß man die Umsetzung in Gegenwart von einer Mischung aus ortho-Borsäure und Weinsäure durchführt.

4. Verfahren zur Herstellung von dl-α-Tocopherol bzw. dl-α-Tocopherylacetat gemäß Anspruch 1, dadurch gekennzeichnet, daß man die Umsetzung in Gegenwart von 0,5 bis 5 mol-% an ortho-Borsäure und 1 bis 10 mol-% an Oxalsäure, Weinsäure oder Citronensäure pro mol Trimethylhydrochinon durchführt.

## Claims

1. A process for preparing dl-α-tocopherol or dl-α-tocopheryl acetate by acid-catalyzed reaction of 2,3,5-trimethylhydroquinone with phytol or isophytol in a solvent at elevated temperature, with or without subsequent esterification of the resulting tocopherol with acetic anhydride, wherein the reaction is carried out in the presence of a mixture of ortho-boric acid on the one hand and oxalic acid, tartaric acid or citric acid on the other hand.

2. A process for preparing dl-α-tocopherol or dl-α-tocopheryl acetate as claimed in claim 1, wherein the reaction is carried out in the presence of a mixture of ortho-boric acid and oxalic acid.

3. A process for preparing dl-α-tocopherol or dl-α-tocopheryl acetate as claimed in claim 1, wherein the reaction is carried out in the presence of a mixture of ortho-boric acid and tartaric acid.

4. A process for preparing dl-α-tocopherol or dl-α-tocopheryl acetate as claimed in claim 1, wherein the reaction is carried out in the presence of from 0.5 to 5 mol % of ortho-boric acid and from 1 to 10 mol % of oxalic acid, tartaric acid or citric acid per mol of trimethylhydroquinone.

## Revendications

1. Procédé pour la préparation du dl-α-tocophérol et de l'acétate de dl-α-tocophéryle par réaction, catalysée par un acide, de la 2,3,5-triméthylhydroquinone avec le phytol ou l'isophytol dans un solvant à température élevée, éventuellement suivie de l'estérification du tocophérol obtenu par l'anhydride acétique, caractérisé par le fait que l'on effectue la réaction en présence d'un mélange d'acide orthoborique d'une part et d'acide oxalique, d'acide tartrique ou d'acide citrique d'autre part.

2. Procédé pour la préparation du dl-α-tocophérol et de l'acétate de dl-α-tocophéryle selon la revendication 1, caractérisé par le fait que l'on effectue la réaction en présence d'un mélange d'acide orthoborique et d'acide oxalique.

3. Procédé pour la préparation du dl-α-tocophérol et de l'acétate de dl-α-tocophéryle selon la revendication 1, caractérisé par le fait que l'on effectue la réaction en présence d'un mélange d'acide orthoborique et d'acide tartrique.

4. Procédé pour la préparation du dl-α-tocophéryle selon la revendication 1, caractérisé par le fait que l'on effectue la réaction en présence de 0,5 à 5 mol % d'acide orthoborique et de 1 à 10 mol % d'acide oxalique, d'acide tartrique ou d'acide citrique, par mol de la triméthylhydroquinone.
